# EUROPÄISCHE PATENTSCHRIFT

(11) **EP 1 049 707 B1**
(45) Veröffentlichungstag und Bekanntmachung des Hinweises auf die Patenterteilung: **07.05.2008**
(21) Anmeldenummer: 99903645.2
(22) Anmeldetag: 19.01.1999
(51) Int. Cl.: C07H 15/04, C12P 19/02, A61K 31/7004

(54) **USTILIPIDE, VERFAHREN ZU DEREN HERSTELLUNG SOWIE DEREN VERWENDUNG**
USTILIPIDES, METHOD FOR THE PRODUCTION AND THE USE THEREOF
USTILIPIDES, LEUR PROCEDE DE FABRICATION ET LEUR APPLICATION

(30) Priorität: 23.01.1998 DE 19802450
(43) Veröffentlichungstag der Anmeldung: 08.11.2000
(73) Patentinhaber: Sanofi-Aventis Deutschland GmbH, 65929 Frankfurt am Main (DE)
(72) Erfinder: VÉRTESY, Laszló, D-65817 Eppstein (DE); KURZ, Michael, D-65719 Hofheim (DE); NOELKEN, Gerhard, D-65843 Sulzbach (DE); WINK, Joachim, D-63322 Rödermark (DE)
(86) Internationale Anmeldenummer: PCT/EP1999/000288
(87) Internationale Veröffentlichungsnummer: WO 1999/037658

(56) Entgegenhaltungen:
- US-A- 4 582 823
- SORIENTE A ET AL: "Enzymatic regio- and diastereoselective hydrolysis of peracetylated glycerol- and erythritol-beta-glucosides" BIOORGANIC & MEDICINAL CHEMISTRY LETTERS, Bd. 5, Nr. 20, 19. Oktober 1995, Seite 2321-2324 XP004135257
- DEML, GUENTHER ET AL: "Antibiotics from Basidomycetes. Part VIII. Schizonellin A and B, new glycolipids from Schizonella melanogramma" PHYTOCHEMISTRY (1980), 19(1), 83-7 , XP002104498
- KANEDA, MIYUKI ET AL: "Glycerol glucosides in the Lilium genus. Part 3. Liliosides D and E, tw glycerol glucosides from Lilium japonicum" PHYTOCHEMISTRY (1984), 23(4), 795-8 , XP002104499

## Beschreibung

Die vorliegende Erfindung betrifft neue Wirkstoffe (Ustilipide), die von dem Mikroorganismus Ustilago maydis, FH 2634, DSM 11494 während der Fermentation gebildet werden, Verfahren zu deren Herstellung, deren Verwendung als Arzneimittel, Ustilipide enthaltende Arzneimittel sowie den Mikroorganismus Ustilago maydis, FH 2634, DSM 11494.

Die Schizophrenie (Dementia praecox) ist eine endogene, psychosomatische Erkrankung mit Verlust des Strukturzusammenhanges der Persönlichkeit und mit Spaltung von Denken Affekt und Erleben. Sie beruht auf der Interaktion von psychischen und somatischen Faktoren. Die Schizophrenie geht mit schweren Denkstörungen, Antriebsstörungen, Wahn, Haluzinationen und Ich-Erlebnisstörungen einher. Die Behandlung der Schizophrenie erfolgt derzeit hauptsächlich durch Verabreichen von Neuroleptika sowie durch Psychotherapie. Sie ist derzeit noch nicht oder nur eingeschränkt heilbar. Wegen der völlig unzureichenden Therapierbarkeit dieser schweren Erkrankung werden neue Medikamente, die zur Behandlung der Schizophrenie geeignet sind, dringend benötigt.

Aus US-A-4 582 823 sind Di-Phenyl-Alkyladenosine und Di-Phenyl-2-Aminoalkyladenosine und deren Verwendung zur Behandlung von Dementia praecox bekannt.

Es wurde nun gefunden, daß der Stamm, Ustilago maydis, FH 2634, DSM 11494 neue, hoch aktive Wirkstoffe zu bilden vermag, die sich zur Behandlung der Schizophrenie oder anderere, durch Dysfunktion des Dopamin-Stoffwechsels hervorgerufenen Krankheiten eignen, und die auch gut verträglich sind.

Gegenstand der Erfindung sind demzufolge die von dem Stamm Ustilago maydis DSM 11494 gebildeten Wirkstoffe (Ustilipide) sowie deren physiologisch verträglichen Salze, Ester und offensichtliche chemische Äquivalente.

Die Erfindung betrifft somit Verbindungen der Formel 1 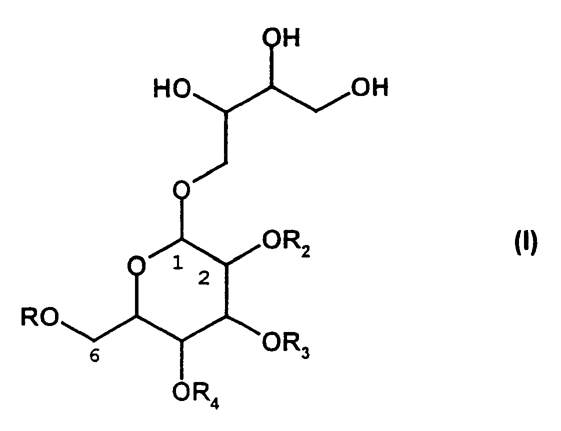 wobei
- R₂, R₃ und R₄: unabhängig voneinander Acylreste mit 2-25 C-Atomen, vorzugsweise mit 2-20 C-Atomen bedeuten, die unsubstituiert sind oder unabhängig voneinander mit 1, 2 oder 3 (C₆-C₁₂)-Arylresten substituiert sind; und
- R: Wasserstoff oder einen unter R², R³ und R⁴ definierten Rest bedeutet;
wobei für den Fall das R gleich Wasserstoff ist, R² einen Acylrest mit 3-25 C-Atomen, vorzugsweise mit 3-20 C-Atomen bedeutet, der unsubstituiert ist oder mit 1, 2 oder 3 (C₆-C₁₂)-Arylresten substituiert ist;
sowie deren physiologisch verträglichen Salze.

Bevorzugt sind die Verbindungen der Formel I, in denen
- R: Wasserstoff oder ein Acylrest mit 2-25 C-Atomen, vorzugsweise mit 2-20 C-Atomen;
- R²: ein Acylrest mit 4-25 C-Atomen , vorzugsweise mit 4 -20 C-Atomen;
- R³: ein Acylrest mit 10-25 C-Atomen, vorzugsweise mit 15-20 C-Atomen; und
- R⁴: ein Acylrest mit 2-25 C-Atomen , vorzugsweise mit 2 -20 C-Atomen; bedeutet;
sowie deren physiologisch verträglichen Salze.

Bevorzugt sind auch Verbindungen der Formel I, in denen R³ ein Acylrest mit 10-20 C-Atomen, bevorzugt 15-18 C-Atomen bedeutet und R, R¹, R³ und R⁴ jeweils unabhängig voneinander ein Acylrest mit 2-10 C-Atomen, besonders bevorzugt mit 2-6 C-Atomen bedeutet sowie deren physiologisch verträglichen Salze.

Die Acylreste der Verbindungen der Formel I können geradkettig oder verzweigt, gesättigt oder einfach, zweifach, dreifach, vierfach oder fünffach ungesättigt sein.

Unter einem Acylrest mit 2 C-Atomen ist beispielsweise ein Acetylrest zu verstehen.

Beispiele für gesättigte, unverzweigte Acylreste sind ein Essigsäurerest (C = 2), Propionsäurerest (C = 3), Buttersäurerest (C =4), Valeriansäurerest (C = 5), Capronsäurerest (C=6), Önanthsäurerest (C=7), Caprylsäurerest (C = 8), Perlagonsäurerest (C = 9), Caprinsäurerest (C = 10), Undecansäurerest (C=1 11), Laurinsäurerest (C = 1 2), Tridecansäurerest (C = 13), Myristinsäurerest (C = 14), Pentadecansäurerest (C = 15), Palmitinsäurerest (C=16), Margarinsäurerest (C = 17), Stearinsäurerest (C = 18), Nonadecansäurerest (C = 19), Arachinsäurerest (C = 20), Behensäurerest (C = 22), Lignocerinsäurerest (C = 24). Beispiele für gesättigte, verzweigte Acylreste sind ein Isobuttersäurerest (C =4), Isovaleriansäurerest (C=5), Tuberculostearinsäurerest (C = 19).

Beispiele für einfach ungesättigte, unverzweigte Acylreste sind ein Acrylsäurerest (C = 3), Crotonsäurerest (C =4), Palmitoleinsäurerest (C = 16), Ölsäurerest (C = 18), Erucasäurerest (C = 22).

Beispiele für zweifach ungesättigte, unverzweigte Acylreste sind ein Sorbinsäurerest (C = 6) und Linolsäurerest (C = 18).

Beispiele für dreifach ungesättigte, unverzweigte Acylreste sind ein Linolensäurerest (C = 18) oder Eläostearinsäurerest (C = 18).

Beispiel für einen vierfach ungesättigten, unverzweigten Acylrest ist ein Arachidonsäurerest (C = 20).

Beispiel für einen fünffach ungesättigten, unverzweigten Acylrest ist ein Clupanodonsäurerest (C = 22).

Unter (C₆-C₁₂)-Aryl wird beispielsweise Phenyl, Naphtyl oder Biphenyl verstanden. Bevorzugt ist Phenyl.

Die Erfindung betrifft des weiteren
a) eine Verbindung der Formel (II) 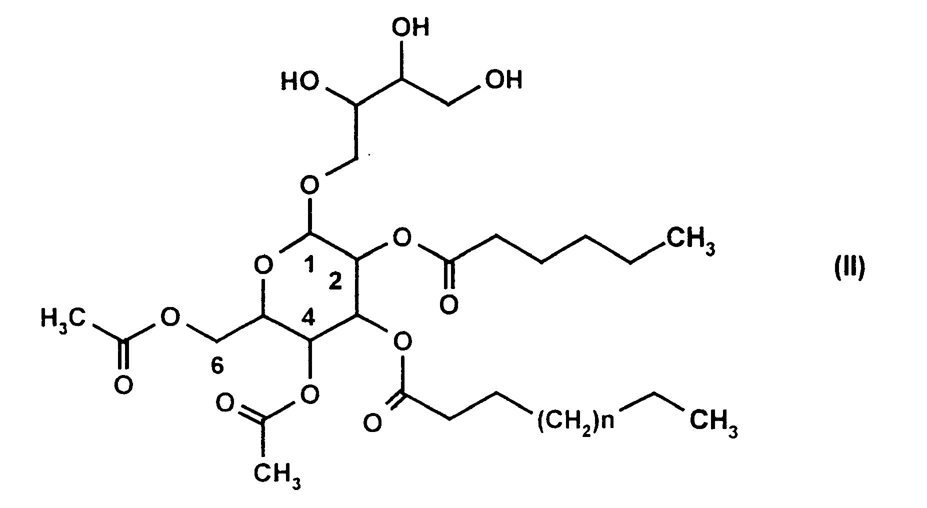 wobei n gleich 11 ist (= Ustilipid A: Summenformel: C₃₆H₆₄O₁₃, MG: 704) sowie deren physiologisch verträglichen Salze;
b) eine Verbindung der Formel (III) 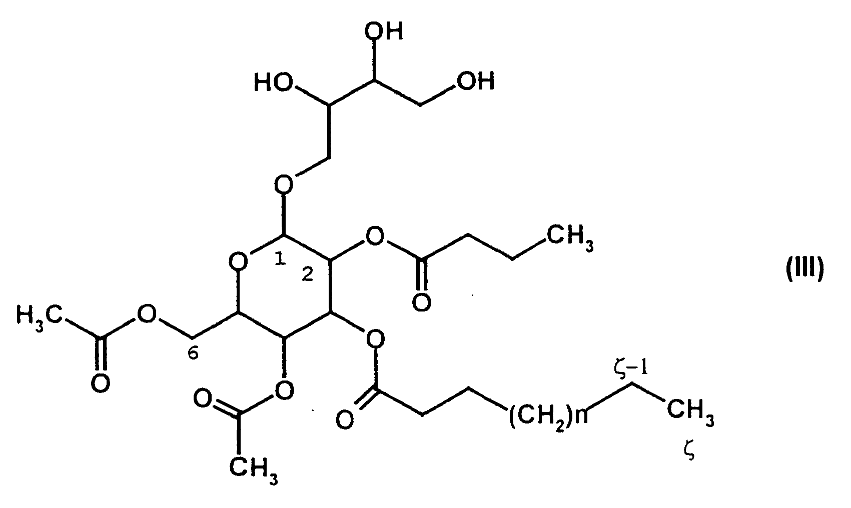 wobei n gleich 11 ist (= Ustilipid B: Summenformel: C₃₄H₆₀O₁₃, MG: 676) sowie deren physiologisch verträglichen Salze; und
c) eine Verbindung der Formel (IV) 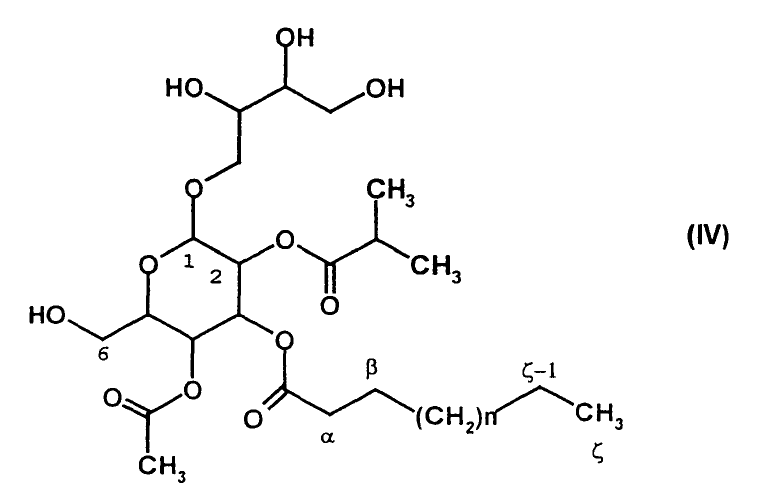 worin n gleich 11 ist (Ustilipid C, Summenformel: C₃₂H₅₈O₁₂, MG: 634), sowie deren physiologisch verträglichen Salze.

Chiralitätszentren in den Verbindungen der Formel I - IV können, wenn nicht anders angegeben, in der R- oder in der S-Konfiguration vorliegen. Die Erfindung betrifft sowohl die optisch reinen Verbindungen als auch Stereoisomerengemische wie Enantiomerengemische und Diasteromerengemische.

Von den erfindungsgemäßen Verbindungen der Formel I -IV, die alle ein Saccharidgerüstes aufweisen,sind solche Verbindungen bevorzugt, in denen die Konfiguration des Saccharid-Gerüsts dem des 1-O-β-D-Manno-pyranosyl-(2R,3S)-erythritol entspricht (Formel V):

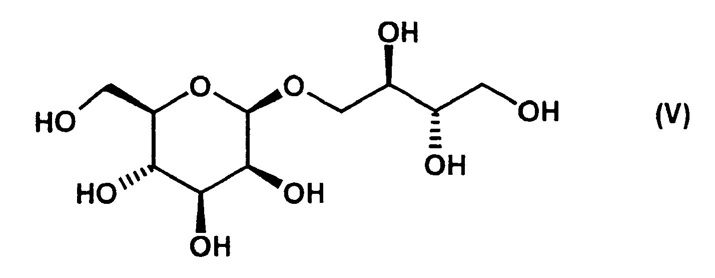

Einige Glycolipide mit einem Mannopyranosyl-erythritol-Gerüst sind schon in der Literatur erwähnt worden (G. Deml et al. Phytochemistry, 19, 83-87, 1980). Die dort beschriebenen Schizonelline A und B, die sich in ihrer Struktur von den erfindungsgemäßen Verbindungen der Formel (I) bis (IV) unterscheiden, sind schwach wirksame Antibiotika mit starker hämolytischer Wirkung.

Die Erfindung betrifft auch ein Verfahren zur Herstellung der genannten Verbindungen der Formel I bis IV, dadurch gekennzeichnet, daß der Mikroorganismus Ustilago maydis FH 2634 (DSM 11494) oder Mutanten und Varianten davon, in einem wäßrigen Nährmedium kultiviert wird und die Zielverbindungen anschließend isoliert und gereinigt werden.

Der Mikroorganismus Ustilago maydis FH 2634 (DSM 1 1494) wurde aus einer Erdprobe isoliert. Der genannte Mikroorganismus ist am 14. April 1997 unter den Bedingungen des Budapester Vertrags bei der Deutschen Sammlung von Mikroorganismen und Zellkulturen, Mascheroder Weg 1b, D-38124 Braunschweig unter der Nummer DSM 11494 hinterlegt worden.

Ustilago maydis, DSM 11494 besitzt weißes Luftmycel und graue Sporenketten. Er bildet charakteristische Sporenketten. In einer Nährlösung, die eine Kohlenstoffquelle und eine Stickstoffquelle sowie die üblichen anorganischen Salze enthält, produziert der Ustilago maydis, DSM 11494 die Ustilipide.

Anstelle des Stammes DSM 11494 können auch dessen Mutanten und Varianten eingesetzt werden, soweit sie die erfindungsgemäßen Verbindungen synthetisieren. Solche Mutanten können in an sich bekannter Weise durch physikalische Mittel, beispielsweise Bestrahlung, wie mit Ultraviolett- oder Röntgenstrahlen, oder chemische Mutagene, wie beispielsweise Ethylmethylansulfonat (EMS); 2-Hydroxy-4-methoxy-benzophenon (MOB) oder N-Methyl-N'-nitro-N-nitrosoguanidin (MNNG) erzeugt werden. Das Screening nach Mutanten und Varianten, die die erfindungsgemäßen Verbindungen produzieren, kann durch Bestimmung der biologischen Aktivität der in der Kulturbrühe angehäuften Wirkstoffe, beispielsweise durch Austesten der Dopamin-antagonisierenden Wirkung nach dem unten beschriebenen Verfahren erfolgen.

Als bevorzugte Kohlenstoffquelle für die aerobe Fermentation eignen sich assimilierbare Kohlenhydrate und Zuckeralkohole, wie Glucose, Laktose oder D-Mannit sowie kohlenhydrathaltige Naturprodukte, wie z.B. Malzextrakt. Als stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anorganischen Salzen kann die Nährlösung beispielsweise Chloride, Carbonate, Sulfate oder Phosphate der Alkali- oder Erdalkalimetalle, Eisen, Zink, Kobalt und Mangan enthalten.

Die Bildung der Ustilipide verläuft besonders gut z.B. in einer Nährlösung, die etwa 0.5 bis 5 % Glucose, vorzugsweise 1 bis 2 %, 0.5 bis 5 % Soyamehl, vorzugsweise 1 bis 2 %, Cornsteep, vorzugsweise 0.2 bis 1 %, 0.05 bis 1.0 % CaCO₃ vorzugsweise 0.1 bis 0.5 % und 0.1 bis 2 % NaCl, vorzugsweise 0.2 bis 1 %, jeweils bezogen auf das Gewicht der gesamten Nährlösung enthält.

Die Kultivierung erfolgt aerob, also beispielsweise submers unter Schütteln oder Rühren in Schüttelkolben oder Fermentern, gegebenenfalls unter Einführen von Luft oder Sauerstoff. Die Fermentation kann beispielsweise in Steilbrustflaschen oder Rundkolben verschiedener Volumina, in Glasfermentern oder V₂A-Stahltanks durchgeführt werden. Sie kann in einem Temperaturbereich von etwa 20 bis 35° C, vorzugsweise bei etwa 25 bis 30° C, durchgeführt werden. Der pH-Wert sollte zwischen 3 und 10 liegen, vorteilhaft zwischen 4,5 und 8,5. Man kultiviert den Mikroorganismus unter diesen Bedingungen im allgemeinen über einen Zeitraum von 20 bis 300 Stunden, bevorzugt 24 bis 140 Stunden. Vorteilhaft kultiviert man in mehreren Stufen, d. h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man z. B. indem man ein versportes Mycel in eine Nährlösung überimpft und etwa 20 bis 120 Stunden, bevorzugt 24 bis 72 Stunden, wachsen läßt. Das versporte Mycel kann beispielsweise erhalten werden, indem man den Stamm etwa 1 bis 40 Tage, bevorzugt 3 bis 10 Tage, auf einem festen oder flüssigen Nährboden, beispielsweise Hefe-Malz-Agar oder Kartoffel-Dextrose-Agar, wachsen läßt.

Der Fermentationsverlauf und die Bildung der Ustilipide kann entsprechend dem Fachmann bekannten Methoden, wie z. B. durch Austestung der biologischen Aktivität in Bioassays oder durch chromatographische Methoden wie Dünnschichtchromatographie (DC) oder Hochleistungsflüssigkeitschromatographie (HPLC) verfolgt werden.

Die Ustilipide können sowohl im Mycel als auch im Kulturfiltrat vorkommen, gewöhnlich befindet sich die Hauptmenge in der Zellmasse (Mycel). Es ist deshalb zweckmäßig, diese durch Filtration oder Zentrifugation vom Filtrat zu trennen. Das Filtrat wird mit einem mit Wasser nicht mischbaren Lösungsmittel wie z. B. 1-Butanol, Essigsäureethylester, Chloroform oder ähnlichem extrahiert. Das Mycel wird zweckmäßigerweise mit Methanol oder Aceton extrahiert, es können aber auch die oben genannten mit Wasser nicht mischbaren Lösungsmittel verwendet werden.

Die Extraktionen können in einem weiten pH-Bereich durchgeführt werden, es ist jedoch zweckmäßig, im neutralen Milieu, vorzugsweise zwischen pH 4 und pH 8 zu arbeiten. Die organischen Extrakte können z. B. im Vakuum konzentriert und getrocknet werden.

Eine Methode der Isolierung der Ustilipide ist die Lösungsverteilung in an sich bekannter Weise.

Eine andere Methode der Reinigung ist die Chromatographie an Adsorptionsharzen wie z. B. an Diaion^{®} HP-20 (Mitsubishi Casei Corp., Tokyo), an Amberlite^{®} XAD 7 (Rohm and Haas, USA), an Amberchrom^{®} CG, (Toso Haas, Philadelphia, USA) oder an ähnlichen. Geeignet sind darüber hinaus zahlreiche Reverse Phase-Träger, z. B. RP 18, wie sie z. B. im Rahmen der Hochdruckflüssigkeits-Chromatographie (HPLC) allgemein bekannt geworden sind.

Eine weitere Reinigungsmöglichkeit für die erfindungsgemäßen Verbindung besteht in der Verwendung von sog. Normal-Phasen-Chromatographie-Trägern, wie z. B. Kieselgel oder Al₂O₃ oder anderen in an sich bekannter Weise. Geeignet sind hierfür viele Lösungen und ihre Mischungen, wie z. B. Chloroform / Petrolether / Alkohol-Gemische, denen saure Lösungsmittel wie z. B. Essigsäure hinzugefügt worden sind.

Ein alternatives Isolierungsverfahren ist die Verwendung von Molekularsieben, wie z. B. Fractogel^{®} TSK HW-40, Sephadex^{®} LH-20 und andere, in an sich bekannter Weise.

Neben den Verbindungen der Formel II, III und IV bildet der Stamm Ustilago maydis, DSM 11494, noch weitere, sehr wirksame Verbindungen der Formel I, die sich durch die Fettsäurezusammensetzung und damit durch die Summenformel sowie Molekulargewichte von den Verbindungen der Formel II, III, und IV unterscheiden. Es können die Fettsäuren von C₂ bis C₂₀ nachgewiesen werden, wobei gesättigte und ungesättigte, verzweigte und unverzweigte Carbonsäuren wie beispielsweise Stearinsäure-, Linolsäure-, Ölsäure- oder C-20 Säurereste vorkommen. So werden von dem Stamm Ustilago maydis, DSM 11494, neben den Ustilipiden A, B und C auch wirksame Glycosidester mit Molekulargewichten von 648, 660, 674, 690, 700, 702, 704, 716, 718, 728, 730, 746 sowie 748 produziert. Das Glycosid-Gerüst der aus Kulturen des Ustilago maydis isolierten Ustilipide ist das bereits beschriebene 1-O-β-D-Mannopyranosyl-(2R,3S)-erythritol (Verbindung der Formel V).

Die Verbindungen der Formel 1 können analog den oben beschriebenen Methoden isoliert und gereinigt werden.

Es wurde gefunden, daß die erfindungsgemäßen Verbindungen Dopamin-D2-Rezeptoren und insbesondere Dopamin-D3-Rezeptoren wirksam antagonisieren.

Das Dopamin (3,4 Dihydroxy-phenylethylamin) ist ein Neutrotransmitter, der Stoffwechselvorgänge im Gehirn und damit Hirnfunktionen steuert. Seine Wirkung erfolgt über das Andocken an Dopaminrezeptoren. Es sind bis jetzt verschiedene Dopamin-Rezeptoren beschrieben worden, die die unterschiedlichen Funktionen des zentralen Nervensystems kontrollieren. Die Störung der Dopamin D3 Rezeptorfunktion ist als eine wesentliche Ursache der Schizophrenie erkannt worden (P. Sokoloff, J.-C. Schwartz et al. Nature, 347, 146-151, 1990). Ihre Überfunktion führt zu krankhaften Prozessen und deshalb sind Dopamin D3-Antagonisten von großer medizinischer Bedeutung. Breit wirkende Dopamin-Rezeptorantagonisten, die nicht nur die krankhafte Überfunktion, sondern auch lebensnotwendige Vorgänge mindern, sind jedoch schädlich: sie verursachen unerwünschte Nebenwirkungen (D. S. Kreiss et al. Eur. J. Pharmacol. 277, 209-214, 1995). Besonders geeignet zur Behandlung der Schizophrenie sind die erfindungsgemäßen Verbindungen, die den D3-Dopaminrezeptor selektiv antagonisieren.
Die erfindungsgemäßen Ustilipide hemmen den Dopamin-Rezeptor D3 zum Teil erheblich stärker als den Dopamin-Rezeptor D2 oder andere Rezeptoren und sind deshalb wertvolle neue Agentien zur Behandlung der Schizophrenie.

Es wurde weiter überraschend gefunden, das die erfindungsgemäßen Ustilipide eine nur geringe hämolytische Wirkung aufweisen. Insbesondere die Komponenten, die Fettsäureester höherer Carbonsäuren (C≥10) sind, zeigen bis zu Konzentrationen von 100 mg pro Liter keinen nennenswerten hämolytischen Effekt.

Die vorliegende Erfindung betrifft demzufolge auch die Anwendung der erfindungsgemäßen Verbindungen als Arzneimittel, sowie die Verwendung der betreffenden Verbindungen zur Herstellung von Arzneimitteln zur Behandlung von Arzneimitteln zur Behandlung und/oder Prophylaxe von Schizophrenie, bzw. zur Behandlung von Erkrankungen, die mit einer Störung der Funktion des Dopamin D3 Rezeptors einhergehen.

Offensichtlichen chemischen Äquivalenten der erfindungsgemäßen Verbindungen sind Verbindungen, die einen geringfügigen chemischen Unterschied aufweisen, also die gleiche Wirkung haben oder sich unter milden Bedingungen in die erfindungsgemäßen Verbindungen umwandeln. Zu den genannten Äquivalenten gehören z.B. Ester, Aminoderivate, Komplexe oder Addukte der bzw. mit den erfindungsgemäßen Verbindungen.

Unter physiologisch verträglichen Salzen von Verbindungen der Formel I, II, III oder IV versteht man sowohl deren organische als auch anorganische Salze, wie sie in Remington's Pharmaceutical Sciences (17. Auflage, Seite 1418 (1985)) beschrieben sind. Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Des weiteren betrifft die vorliegende Erfindung Arzneimittel mit einem Gehalt an mindestens einer erfindungsgemäßen Verbindung.

Die erfindungsgemäßen Arzneimittel können enteral (oral), parenteral (intravenös), rektal oder lokal (topisch) angewendet werden. Sie können in Form von Lösungen, Pulvern (Tabletten, Kapseln einschließlich Mikrokapseln), Liposomenpräparaten, Lipid-Komplexen, kolloidalen Dispersionen oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage. Als zweckmäßige Dosierung werden 0.1 - 100 mg/kg Körpergewicht verabreicht. Sie werden zweckmäßig in Dosierungseinheiten verabreicht, die mindestens die wirksame Tagesmenge der erfindungsgemäßen Verbindungen, z. B. 30 - 3000 mg enthalten.

Die Erfindung betrifft weiterhin pharmazeutische Zubereitungen, die eine oder mehrere der erfindungsgemäßen Verbindungen enthalten. Die Arzneimittel können z.B. in Form von pharmazeutischen Präparaten, die man oral, z.B. in Form von Tabletten, Dragees, Hart- oder Weichgelatinekapseln, Lösungen, Emulsionen oder Suspensionen verabreichen kann, verwendet werden. Der Einschluß der Arzneimittel in Liposomen, die gegebenenfalls weitere Komponenten wie Proteine enthalten, ist eine ebenfalls geeignete Applikationsform. Sie können auch rektal z.B. in Form von Suppositorien oder parenteral z.B. in Form von Injektionslösungen verabreicht werden. Für die Herstellung von pharmazeutischen Präparaten können diese Verbindungen in therapeutisch inerten organischen und anorganischen Trägern verarbeitet werden. Beispiele von solchen Trägern für Tabletten, Dragees und Hartgelatinekapseln sind Laktose, Maisstärke oder Derivate davon, Talg und Stearinsäure oder Salze davon. Geeignete Träger für die Herstellung von Lösungen sind Wasser, Polyole, Saccharose, Invertzucker und Glucose. Geeignete Träger für Injektionslösungen sind Wasser, Alkohole, Polyole, Glycerol und pflanzliche Öle. Geeignete Träger für Suppositorien sind pflanzliche und gehärtete Öle, Wachse, Fette und halbflüssige Polyole. Die pharmazeutischen Präparate können auch Konservierungsmittel, Lösemittel, Stabilisierungsmittel, Netzmittel, Emulgatoren, Süßstoffe, Farbstoffe, Geschmacksmittel, Salze zur Veränderung des osmotischen Drucks, Puffer, Überzugsmittel, Antioxidantien, sowie ggf. andere therapeutische Wirkstoffe enthalten.

Die Erfindung betrifft auch ein Verfahren zur Herstellung eines erfindungsgemäßen Arzneimittels, das dadurch gekennzeichnet ist, daß man mindestens eine der erfindungsgemäßen Verbindungen mit einem pharmazeutisch geeigneten und physiologisch verträglichen Träger und gegebenenfalls weiteren geeigneten Wirk, Zusatz- oder Hilfsstoffen in eine geeignete Darreichungsform bringt.

Bevorzugte Verabreichungsformen sind orale und topische Applikationen, lokale Applikationen wie beispielsweise mit Hilfe eines Katheters oder aber auch Injektionen.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung oder daraus abgeleiteter chemischer Derivate enthält. Bei festen Dosierungseinheiten wie Tabletten, Kapseln und Suppositorien kann diese Dosis bis zu etwa 200 mg, bevorzugt jedoch etwa 0,1 bis 100 mg, und bei Injektionslösungen in Ampullenform bis zu etwa 200 mg, vorzugsweise aber etwa 0,5 bis 100 mg, pro Tag betragen.

Die zu verabreichende Tagesdosis ist abhängig vom Körpergewicht, Alter, Geschlecht und Zustand des Säugers. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber in mehreren kleineren Dosierungseinheiten als auch durch Mehrfachgabe unterteilter Dosen in bestimmten Intervallen erfolgen.

Die folgenden Beispiele sollen der näheren Erläuterung der Erfindung dienen, ohne die Breite der Erfindung in irgendeiner Weise begrenzen zu wollen.

### Beispiel 1 : Herstellung einer Sporensuspension des Produzentenstammes, Ustilago maydis, FH 2634, DSM 11494.

100 ml Nährlösung (20 g Malzextrakt, 2 g Hefeextrakt, 10 g Glucose, 0,5 g (NH₄)₂HPO₄ in 1 L Leitungswasser, pH-Wert vor der Sterilisation: 6,0) in einem 500 ml sterilen Erlenmeyerkolben werden mit dem Stamm Ustilago maydis, FH 2634, DSM 11494, beimpft und 72 Stunden bei 25°C und 140 UpM auf einer rotierenden Schüttelmaschine inkubiert. Anschließend werden 120 ml Kulturflüssigkeit in einem sterilen 500 ml Erlenmeyerkolben mit dem Nährboden Hafermehlinfus, 2,0 g/L, dem zusätzlich 15 g Agar/L zur Verfestigung zugegeben wurde, gleichmäßig verteilt und dekantiert. Die Kulturen werden 10 bis 14 Tage bei 25 C inkubiert. Die nach dieser Zeit entstandenen Sporen eines Kolbens werden mit 500 ml entionisiertem Wasser, das einen Tropf eines handelsüblichen nichtionischen Tensids (z.B. ^{®}Triton X 100, Fa. Serva) enthält, abgeschwemmt, sofort weiterverwendet oder bei -22° C in 50 % Glycerin oder in 10 % Dimethylsulfoxid bei -140° C aufbewahrt.

### Beispiel 2: Herstellung einer Kultur bzw. einer Vorkultur des Produzentenstammes im Erlenmeyerkolben.

Ein steriler 500 ml Erlenmeyerkolben mit 100 ml der im Beispiel 1 beschriebenen Nährlösung wird mit einer auf einem Schrägröhrchen gewachsenen Kultur oder mit 0,2 ml Sporensuspension angeimpft und auf einer Schüttelmaschine im Dunkeln bei 140 UpM und 25° C inkubiert. Die maximale Produktion der Verbindungen der Formel I bis IV ist nach ca. 72 Stunden erreicht. Zum Animpfen von 10 und 100 L Fermentern genügt eine 72 Stunden alte Submerskultur (Animpfmenge ca. 5 %) aus der gleichen Nährlösung.

### Beispiel 3: Herstellung der Ustilipide.

Ein 10 L Fermenter wird unter folgenden Bedingungen betrieben:

| | | |
|---|---|---|
| Nährmedium: | Malzextrakt | 20 g/L |
| | Hefeextrakt | 2 g/L |
| | Glucose | 10 g/L |
| | (NH₄)₂HPO₄ | 2 g/L |
| | pH 6.0 (vor dem Sterilisieren) | |
| Inkubationszeit: | 48 oder 72 Stunden, | |
| Inkubationstemperatur: | 25° C, | |
| Rührergeschwindigkeit: | 200 UpM, | |
| Belüftung: | 5 L Luft/min. | |

Durch wiederholte Zugabe weniger Tropfen ethanolischer Polyollösung kann die Schaumbildung unterdrückt werden. Das Produktionsmaxiumum wird nach 48 Stunden erreicht.

### Beispiel 4: Isolierung des Ustilipid-Komplexes.

9 L der nach Beispiel 3 gewonnenen Kulturlösung werden abzentrifugiert und die Zellmasse (^{~}1,3 L) zweimal mit je 3 L Methanol ausgerührt. Die vereinigten Extrakte werden im Vakuum eingeengt, getrocknet und die Trockenmasse (48 g) mit 25 % Isopropanol/75 % Wasser gelöst, auf eine 3 L fassende, mit dem Adsorptionsharz MCl Gel^{®} CHP20P gefüllte Säule aufgetragen. Säulenmaße: Breite x Höhe: 11,3 cm x 30 cm. Eluiert wird mit einem Lösungsmittel-Gradienten von 25 % Isopropanol in Wasser bis 100 % Isopropanol und der Säulenausfluß in Fraktionen je 2 L aufgefangen.

Die Ustilipid-haltigen Fraktionen, werden gesammelt und im Vakuum konzentriert sowie gefriergetrocknet (8 g).

### Beispiel 5: Anreicherung der Ustilipid-Komponenten.

5g des nach Beispiel 4 gewonnenen Produktes werden auf eine 3 Liter fassende mit Fractogel^{®} TSK HW-40 s gefüllte Säule (Breite x Höhe = 10 cm x 50 cm) aufgetragen. Das Laufmittel Methanol wird mit einer Flußrate von 50 ml pro Minute über die Säule gepumpt und der Säulenausfluß fraktioniert (65 ml) aufgefangen. In den Fraktionen 18 bis 32 befindet sich hauptsächlich der Ustilipid-Komplex (1,8 g). Sie werden zusammengefaßt und im Vakuum vom Lösemittel befreit.

### Beispiel 6: Trennung der Ustilipid-Komponenten.

1,8 g des Ustilipid-Komplexes, gewonnen nach Beispiel 5, werden in Chloroform gelöst und auf eine, mit Kieselgel gefüllte 290 mL fassende Säule aufgetragen. Die Elution erfogt zunächst mit reinem Chloroform, anschliessend mit Chloroform-Mischungen mit ansteigendem Methanol-Anteilen von 0 bis 5% Alkohol. Fraktioniert wird in 50 mL Portionen. Die Analyse der Fraktionen geschieht an Hand der Dopamin D3 / D2 - Hemmteste sowie dünnschichtchromatographisch mit dem α-Naphthol/Schwefelsäure-Reagenz. Die hauptsächlich Ustilipid A- bzw. B- bzw. C-haltigen Fraktionen werden jeweils zusammengefaßt und im Vakuum konzentriert. Es resultieren 105 mg Ustilipid A, 230 mg Ustilipid B sowie 170 mg Ustilipid C-Kompononte in > 50%-iger Reinheit.

### Beispiel 7: Endreinigung der Ustilipid-Komponenten.

Die angereicherten Ustilipide A (104mg ), B (210 mg) sowie C (165 mg), gewonnen nach Beispiel 6, werden jeweils auf einer Nucleosil^{®} 12C₁₈AB-HPLC-Säule (Breite x Höhe = 3,2 cm x 25 cm) im Gradientenverfahren mit 50 % bis 80 % Acetonitril in 0,05% Trifluoressigsäure aufgetrennt. Die durch Dünnschichtchromatographie auf Reinheit untersuchten Fraktionen werden entsprechend zusammengefaßt, im Vakuum eingeengt und gefriergetrocknet. Sie ergeben 63 mg Ustilipid A in 96 %iger Reinheit,162 mg Ustilipid B in 94 %iger Reinheit und 112 mg Ustilipid C in 95 %iger Reinheit.

Die physikalisch-chemischen sowie spektroskopischen Eigenschaften der Ustilipide lassen sich wie folgt zusammenfassen:
Ustilipid A (Verbindung der Formel II):

| | |
|---|---|
| Aussehen: | farblose, in Alkoholen und anderen organischen Lösungsmitteln lösliches Öl. Stabil in neutralem und mild saurem Milieu, jedoch unbeständig in alkalischer Lösung. |
| Summenformel: | C₃₆H₆₄O₁₃ |
| Molekulargewicht: | 704 |
| ¹H- und ¹³C-NMR: siehe Tabelle 1 | |
| UV-Absorption: | Endabsorption. |
| [a]_{D} = -34° (c = 0.5 in Chloroform) | |

Ustilipid B (Verbindung der Formel III):

| | |
|---|---|
| Aussehen: | farblose, in Alkoholen und anderen organischen Lösungsmitteln lösliches Öl. Stabil in neutralem und mild saurem Milieu, jedoch unbeständig in alkalischer Lösung. |
| Summenformel: | C₃₄H₆₀O₁₃ |
| Molekulargewicht: | 676 |
| ¹H- und ¹³C-NMR: siehe Tabelle 2 | |
| UV-Absorption: | Endabsorption. |
| [a]_{D} - -33° (c = 0.5 in Chloroform) | |

Ustilipid C (Verbindung der Formel IV)

| | |
|---|---|
| Aussehen: | farblose, in Alkoholen und anderen organischen Lösungsmitteln lösliches Öl. Stabil in neutralem und mild saurem Milieu, jedoch unbeständig in alkalischer Lösung. |
| Summenformel: | C₃₂H₅₈O₁₂ |
| Molekulargewicht: | 634 |
| ¹ H- und ¹³C-NMR: siehe Tabelle 3. | |
| UV-Absorption: | Endabsorption. |
| [a]_{D} = -38° ( c = 0.5 in Chloroform) | |

**Tabelle 1:**

| ¹H- und ¹³C-NMR: Chemische Verschiebungen von Ustilipid A in CDCl₃ bei 300K | | |
|---|---|---|
| | ¹H | ¹³C |
| 1 | 4.72 | 100.00 |
| 2 | 5.50 | 69.30 |
| 3 | 5.06 | 71.35 |
| 4 | 5.24 | 66.60 |
| 5 | 3.70 | 73.20 |
| 6 | 4.25/4.19 | 63.06 |
| 1α | 3.99/3.82 | 72.99 |
| 1β | 3.74 | 71.86 |
| 1γ | 3.65 | 72.55 |
| 1δ | 3.75 | 64.27 |
| 2C' | - | 174.16 |
| 2α | 2.43 | 34.80 |
| 2β | 1.65 | 25.37 |
| 2γ | 1.34 | 31.81 |
| 2δ | 1.34 | 22.97 |
| 2ε | 0.90 | 14.55 |
| 3C' | - | 173.42 |
| 3α | 2.21 | 34.70 |
| 3β | 1.53 | 25.39 |
| 3γ | 1.24 | 29.72 |
| 3δ-3ξ-3 | 1.36-1.21 | 30.35-29.90 |
| 3ξ-2 | 1.28 | 32.59 |
| 3ξ-1 | 1.28 | 23.35 |
| 3ξ | 0.87 | 14.78 |
| 4C' | | 170.14 |
| 4-Me | 2.03 | 21.33 |
| 6C' | - | 171.46 |
| 6-Me | 2.09 | 21.39 |

**Tabelle 2:**

| ¹H- und ¹³C-NMR: Chemische Verschiebungen von Ustilipid B in CDCl₃ bei 300K | | |
|---|---|---|
| | ¹H | ¹³C |
| 1 | 4.73 | 99.97 |
| 2 | 5.50 | 69.33 |
| 3 | 5.06 | 71.38 |
| 4 | 5.24 | 66.58 |
| 5 | 3.70 | 73.13 |
| 6 | 4.25/4.18 | 63.04 |
| 1α | 3.99/3.79 | 72.92 |
| 1β | 3.73 | 71.83 |
| 1γ | 3.63 | 72.59 |
| 1δ | 3.71 | 64.21 |
| 2C' | - | 173.99 |
| 2α | 2.41 | 36.71 |
| 2β | 1.68 | 19.18 |
| 2γ | 0.98 | 14.20 |
| 2δ | - | - |
| 3C' | - | 173.41 |
| 3α | 2.20 | 34.68 |
| 3β | 1.52 | 25.35 |
| 3γ-3ξ-1 | 1.36-1.21 | 30.32-29.68 |
| 3ζ | 0.86 | 14.75 |
| 4C' | - | 170.15 |
| 4-Me | 2.02 | 21.31 |
| 6C' | - | 171.44 |
| 6-Me | 2.09 | 21.37 |

**Tabelle 3:**

| ¹H- und ¹³C-NMR: Chemische Verschiebungen von Ustilipid C in CDCl₃ bei 300K | | |
|---|---|---|
| | ¹H | ¹³C |
| 1 | 4.79 | 99.75 |
| 2 | 5.49 | 69.40 |
| 3 | 5.09 | 71.52 |
| 4 | 5.16 | 66.95 |
| 5 | 3.56 | 75.51 |
| 6 | 3.67 | 62.1 |
| 1α | 4.02/3.77 | 72.50 |
| 1β | 3.76 | 71.71 |
| 1γ | 3.64 | 72.86 |
| 1δ | 3.72 | 64.08 |
| 2C' | - | 177.56 |
| 2α | 2.68 | 34.63 |
| 2β | 1.21 | 19.96 |
| 2γ | 1.18 | 19.49 |
| 3C' | - | 173.41 |
| 3α | 2.20 | 34.69 |
| 3β | 1.52 | 25.37 |
| 3γ-3ζ-1 | 1. 35-1.22 | 30.35-29.72 |
| 3ζ | 0.87 | 14.77 |
| 4C' | - | 170.76 |
| 4-Me | 2.04 | 21.37 |

### Beispiel 8: Bestimmung der biologischen Aktivität

Die Ustlipide antagonisieren die Dopamin-D2 und -D3-Rezeptoren wirksam. Ein Maß ihrer Aktivität ist der IC₅₀ -Wert, der die Inhibitor-Konzentration angibt, bei der die Geschwindigkeit der normalen enzymatischen Reaktion (oder Rezeptor-Antagonisierung) um 50 % reduziert wird.

Für die Ustilipide A, B und C wurden folgende Hemmkonstanten bestimmt:

| | Dopamin D3 IC₅₀ | Dopamin D2 IC₅₀ : |
|---|---|---|
| Ustilipid A : | 5 µg / ml | 30 µg / ml |
| Ustilipid B : | 7 µg / ml | 16 µg / ml |
| Ustilipid C : | 12 µg / ml | 9 µg / ml |

### Methode zur Bestimmung der IC₅₀-Werte:

Die D₃- und D₂Lang -Gene wurden aus einer menschlichen cDNA-Bibliothek isoliert und stabil in CHO-Zellen (CHO = Chinese hamster ovary) transfiziert. Membranfraktionen werden aus einzelnen, hoch exprimierenden Klonen gewonnen.
Der Bindungstest wird in 96-Loch Platten für 60 Minuten bei Raumtemperatur durchgeführt. Der Reaktionsansatz enthält 50 µl [N-Methyl-³H]Spiroperidol, 50 µl Testsubstanz oder S(-)-Eticlopirid zur unspezifischen Bindung und 100 µl Membrane. Der Test wird gestoppt durch schnelle Filtration durch einen GF/B Filter in einen Skatron 96 Zellsammler. Die Filter werden zerschnittten, in Behälter gegeben (T-trays, Wallace) und die Radioaktivität wird in einem Scintillationszähler bestimmt.
Die spezifische Bindung ist definiert als die Differenz zwischen der Gesamtbindung und der Bindung in Gegenwart von 10µM S(-)-Eticloprid. Die Gesamtbindung entspricht ungefähr 10% der Zählimpulse der ingesamt eingesetzten Liganden.
Die Hemmung der Bindung durch eineTestsubstanz wird in Korrelation zur Kontrollreaktion berechnet. Die Analyse der Daten erfolgte mit Hilfe des Software Pakets LIGAND von McPherson, Munson & Rodbard, Elsevier-BIOSOFT.

### Beispiel 9:

Von den Schizonellinen A und B, die eine ähnliche Struktur wie die erfindungsgemäßen Verbindungen aufweisen, ist bekannt, daß sie eine sehr starke hämolytische Wirkung aufweisen (G. Deml et al. Phytochemistry, 19, 83-87, 1980), wodurch diese Verbindungen für die parenterale Applikation ungeeignet sind. Bei der Anwendung der Schizonellinen tritt die tötliche 100 % -ige Hämolyse bereits bei Konzentrationen von 10 bzw. 30 µg /ml ein. Eine Bestimmung der hämolytischen Wirkung der Ustilipide A, B und C ergab folgende Ergebnisse, wobei das Maß der Hämolyse in % der zerplatzten Zellen angegeben ist.

| | Ustilipid C | Ustilipid B | Ustilipid A |
|---|---|---|---|
| 125 µg / ml | 0,8% | 0,4% | 0,1% |
| 62,5 µg / ml | 0,5% | 0,0% | 0,0% |
| 32 µg / ml | 0,4% | 0,0% | 0,0% |
| 16 µg / ml | 0,4% | 0,0% | 0,0% |
| 8 µg / ml | 0,0% | 0.0% | 0,0% |

Die Hämolyse-Versuche wurden mit menschlichen Erythrocyten durchgeführt.

### Bestimmung der in-vitro Hämolyse:

Zur Messung der hämolytischen Aktivität wird frisch entnommenes, venöses Blut vom Mensch, Rhesusaffen oder Beaglehund verwendet. Das Blut wird in heparinisierten Röhrchen gesammelt und in Aliquots von 200 µl auf 12 Polyethylenröhrchen verteilt. Ein Aliquot wird mit 200 µl destilliertem Wasser versetzt und dient als 100 % Standard, ein anderes wird mit 200 µl physiologischer Kochsalzlösung (0,9 % NaCl) gemischt (0 % Standard). Je 200 µl von Substanzverdünnungen in physiologischer Kochsalzlösung zu 1600, 800, 400, 200, 100, 50, 25, 12,5, 6,25 und 2,125 µl werden auf die übrigen Röhrchen verteilt. Sämtliche Röhrchen werden vorsichtig geschwenkt und dann für 3 Stunden bei 37°C inkubiert. Anschließend wird der 100 % Standard mit 5 ml destilliertem Wasser, die übrigen Röhrchen mit je 5 ml physiologischer Kochsalzlösung aufgefüllt und 5 Minuten bei 700 g zentrifugiert.

Die Hämolyse wird durch Messung der Absorption des Überstandes in einem Spektralphotometers bei einer Wellenlänge von 540 nm bestimmt. Die Absorption des Standards mit kompletter Hämolyse wird als 100 % gesetzt. Die Absorption der Testpräparatverdünnungen und des 0 % Standards werden gemessen und als Prozent der maximal induzierbaren Hämolyse angegeben.

## Patentansprüche

1. Verbindungen der Formel I, 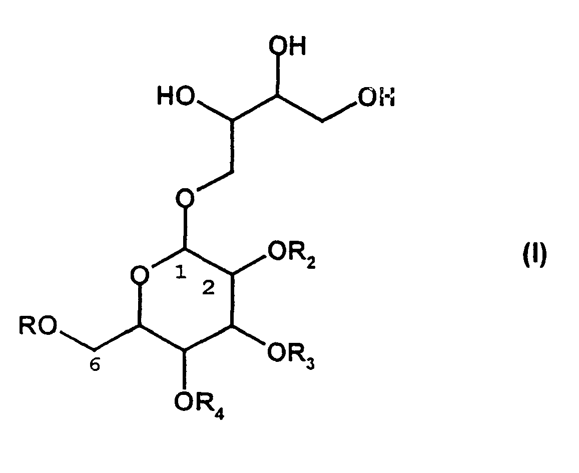 in der bedeuten:
R₂, R₃ und R₄ unabhängig voneinander Acylreste mit 2-25 C-Atomen, die unsubstituiert sind oder unabhängig voneinander mit 1, 2 oder 3 (C₆-C₁₂)-Arylresten substituiert sind; und
R Wasserstoff oder einen unter R², R³ und R⁴ definierten Rest;
wobei für den Fall das R gleich Wasserstoff ist, R² einen Acylrest mit 3-25 C-Atomen, der unsubstituiert ist oder mit 1, 2 oder 3 (C₆-C₁₂)-Arylresten substituiert ist;
sowie deren physiologisch verträglichen Salze.

2. Verbindung der Formel I nach Anspruch 1 in der bedeuten:
R Wasserstoff oder ein Acylrest mit 2-25 C-Atomen;
R² ein Acylrest mit 4-25 C-Atomen ;
R³ ein Acylrest mit 10-25 C-Atomen; und
R⁴ ein Acylrest mit 2-25 C-Atomen; bedeutet;
sowie deren physiologisch verträglichen Salze.

3. Verbindung der Formel (II) 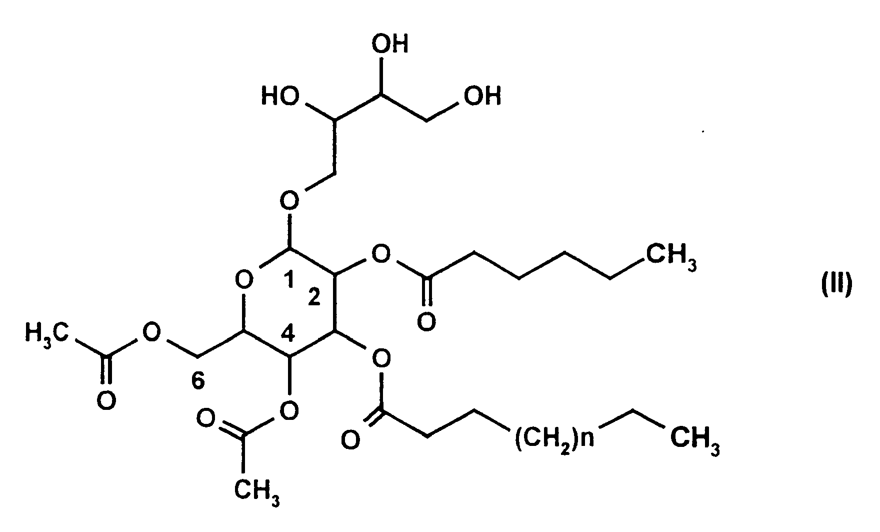 wobei n gleich 11 ist;
sowie deren physiologisch verträglichen Salze.

4. Verbindung der Formel (III) 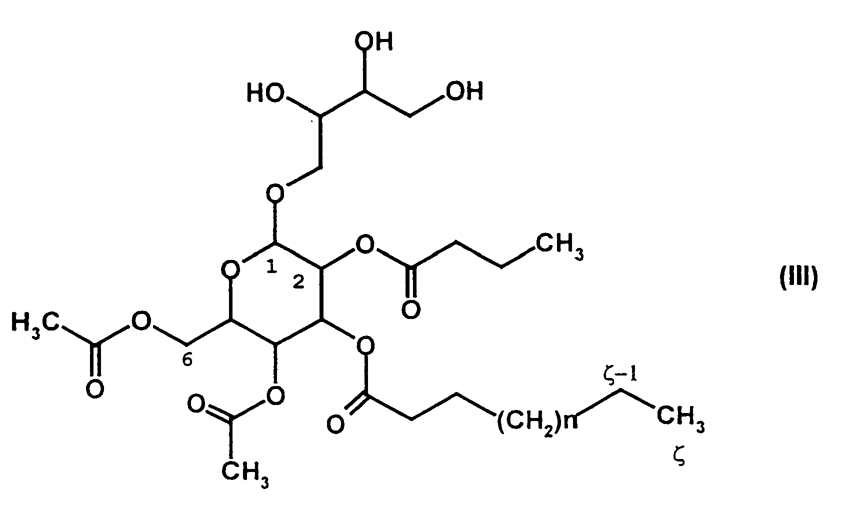 wobei n gleich 11 ist;
sowie deren physiologisch verträglichen Salze.

5. Verbindung der Formel (IV) 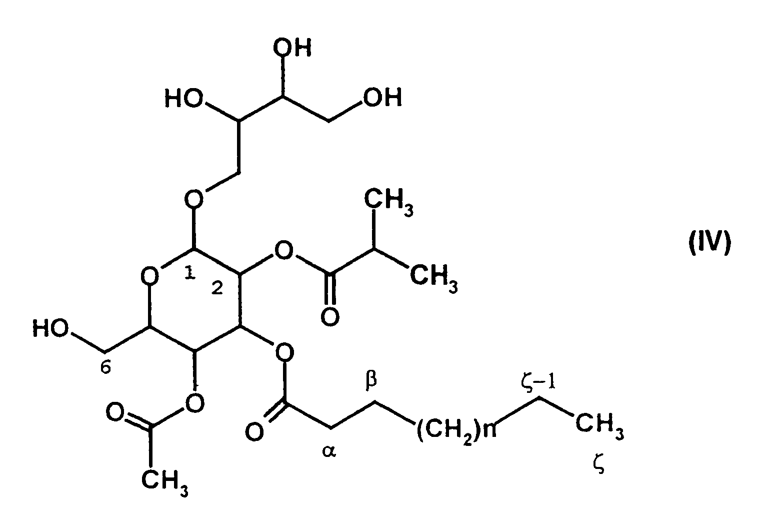 worin n gleich 11 ist;
sowie deren physiologisch verträglichen Salze.

6. Verbindung der Formel I, II, III oder IV oder ein physiologisch verträgliches Salz davon gemäß einem oder mehreren der Ansprüche 1-5, herstellbar indem der Mikroorganismus Ustilago maydis DSM 11494 bzw. einer seiner Varianten oder Mutanten unter geeigneten Bedingungen fermentiert wird, eine oder mehrere der Ustilipide isoliert werden und diese gegebenenfalls in physiologisch verträglichen Salze überführt werden.

7. Verfahren zur Herstellung einer Verbindung der Formel I, II, III oder IV oder ein physiologisch verträgliches Salz davon gemäß einem oder mehreren der Ansprüche 1-5, **dadurch gekennzeichnet, daß** der Mikroorganismus Ustilago maydis DSM 11494 bzw. einer seiner Varianten oder Mutanten unter geeigneten Bedingungen fermentiert wird, eine oder mehrere der Ustilipide isoliert werden und diese gegebenenfalls in physiologisch verträgliche Salze überführt werden.

8. Verfahren gemäß Anspruch 7, **dadurch gekennzeichnet, daß** die Fermentation unter aeroben Bedingungen bei einer Temperatur zwischen 20 und 35°C und bei einem pH zwischen 3 und 10 durchgeführt wird.

9. Verbindung der Formel I, II, III oder IV oder ein physiologisch verträgliches Salz davon gemäß einem oder mehreren der Ansprüche 1-5 zur Anwendung als Arzneimittel.

10. Verbindung der Formel 1, 11, 111 oder IV oder ein physiologisch verträgliches Salz davon gemäß einem oder mehreren der Ansprüche 1 - 5 zur Verwendung als Dopamin-D2 und/oder Dopamin-D3 Rezeptorantagonist.

11. Verwendung von einer Verbindung der Formel I, II, III oder IV oder ein physiologisch verträgliches Salz davon gemäß einem oder mehreren der Ansprüche 1-5 zur Herstellung von Arzneimitteln zur Behandlung von Schizophrenie oder durch Dysfunktion des Dopamin-Stoffwechsels hervorgerufenen Erkrankungen.

12. Arzneimittel mit einem Gehalt an mindestens einer Verbindung der Formel I, II, III oder IV oder ein physiologisch verträgliches Salz davon gemäß einem oder mehreren der Ansprüche 1-5.

13. Verfahren zur Herstellung von Arzneimitteln gemäß Anspruch 12, **dadurch gekennzeichnet, daß** man mindestens eine Verbindung der Formel I, 11, III oder IV oder ein physiologisch verträgliches Salz davon gemäß einem oder mehreren der Ansprüche 1-5 den mit geeigneten Hilfs- und/oder Trägerstoffen in eine geeignete Darreichungsform bringt.

14. Mikroorganismus Ustilago maydis DSM 11494.

## Claims

1. Compounds of the formula I 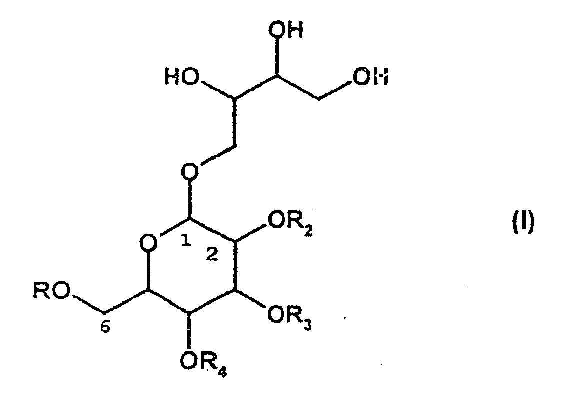 where
R₂, R₃ and R₄ are, independently of one another, acyl radicals with 2-25 carbon atoms, which are unsubstituted or are substituted, independently of one another, by 1, 2 or 3 (C₆-C₁₂) -aryl radicals; and
R is hydrogen or a radical defined under R², R³ and R⁴;
where in the case where R is hydrogen, then R² is an acyl radical with 3-25 carbon atoms, which is unsubstituted or substituted by 1, 2 or 3 (C₆-C₁₂)-aryl radicals;
and also their physiologically tolerated salts.

2. Compound of the formula I according to claim 1, wherein:
R is hydrogen or an acyl radical with 2-25 carbon atoms;
R² is an acyl radical with 4-25 carbon atoms;
R³ is an acyl radical with 10-25 carbon atoms; and
R⁴ is an acyl radical with 2-25 carbon atoms;
and also its physiologically tolerated salts.

3. Compound of the formula (II) 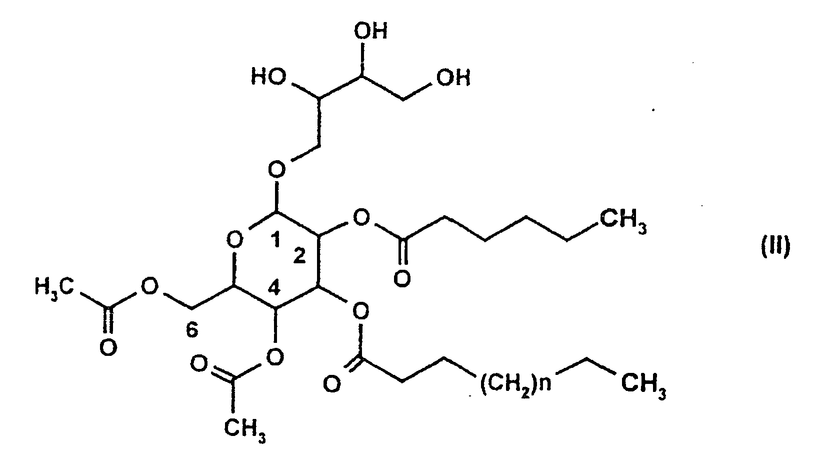 where n is 11;
and also its physiologically tolerated salts.

4. Compound of the formula (III) 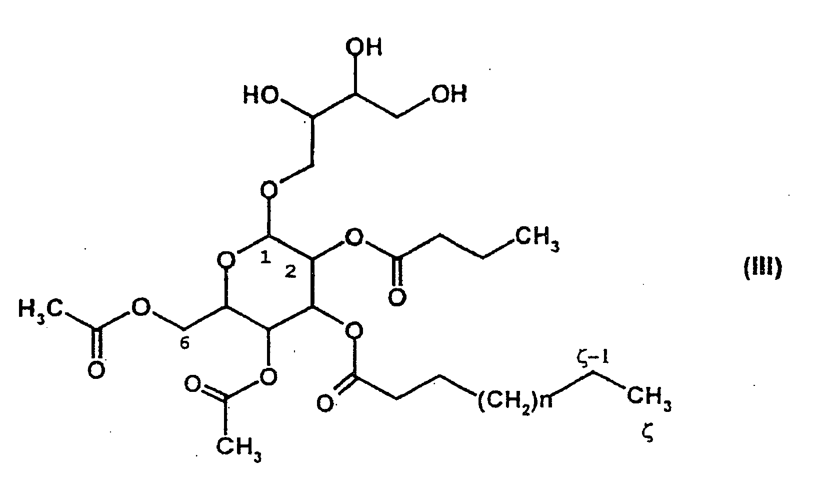 where n is 11;
and also its physiologically tolerated salts.

5. Compound of the formula (IV) 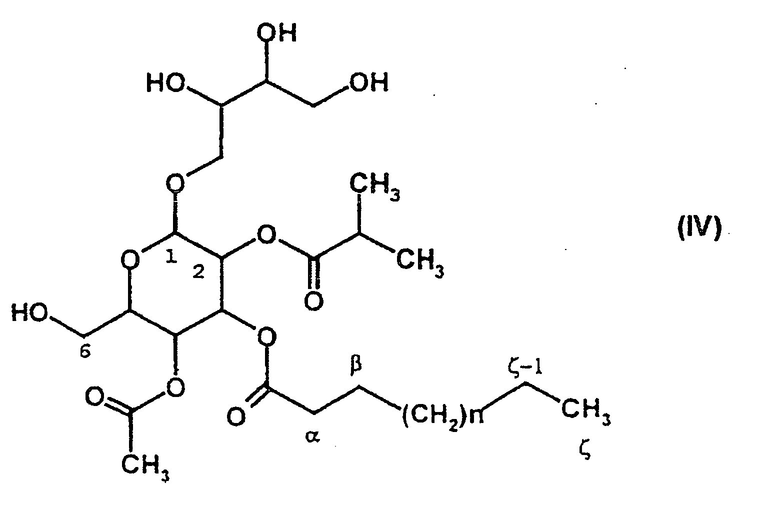 where n is 11;
and also its physiologically tolerated salts.

6. Compound of the formula I, II, III or IV or a physiologically tolerated salt thereof according to one or more of Claims 1-5, obtainable by fermenting the microorganism Ustilago maydis DSM 11494 or one of its variants or mutants under suitable conditions, isolating one or more of the ustilipides, and converting these where appropriate into physiologically tolerated salts.

7. A method of producing a compound of the formula I, II, III or IV or a physiologically tolerated salt thereof according to one or more of Claims 1-5, **characterized in that** it comprises fermenting the microorganism Ustilago maydis DSM 11494 or one of its variants or mutants under suitable conditions, isolating one or more of the ustilipides, and converting these where appropriate into physiologically tolerated salts.

8. A method according to Claim 7, **characterized in that** the fermentation is carried out under aerobic conditions at a temperature between 20 and 35°C and at a pH between 3 and 10.

9. Compound of the formula I, II, III or IV or a physiologically tolerated salt thereof according to one or more of Claims 1-5 for use as a pharmaceutical.

10. Compound of the formula I, II, III or IV or a physiologically tolerated salt thereof according to one or more of Claims 1-5 for use as a dopamine-D2 and/or dopamine-D3 receptor antagonist.

11. The use of a compound of the formula I, II, III or IV or of a physiologically tolerated salt thereof according to one or more of Claims 1-5 in the manufacture of pharmaceuticals for treating schizophrenia or disorders caused by dysfunction of the dopamine metabolism.

12. Pharmaceuticals comprising at least one compound of the formula I, II, III or IV or a physiologically tolerated salt thereof according to one or more of Claims 1-5.

13. A method of producing pharmaceuticals according to Claim 12, **characterized in that** at least one compound of the formula I, II, III or IV or a physiologically tolerated salt thereof according to one or more of Claims 1-5 is brought into a suitable administration form by means of suitable excipient and/or carrier materials.

14. The microorganism Ustilago maydis DSM 11494.

## Revendications

1. Composés de formule I 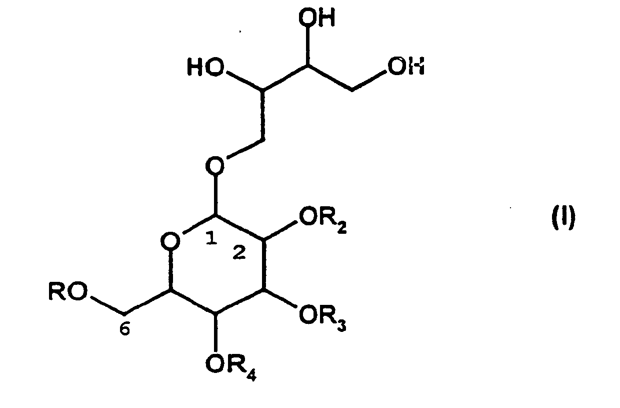 dans laquelle
R₂, R₃ et R₄ signifient, indépendamment l'un de l'autre, des radicaux acyle comprenant 2-25 atomes de carbone, qui sont non substitués ou substitués, indépendamment l'un de l'autre, par 1, 2 ou 3, radicaux aryle en C₆-C₁₂ et
R signifie hydrogène ou un radical défini sous R², R³ et R⁴ ;
où, dans le cas où R représenterait hydrogène, R² représente un radical acyle comprenant 3-25 atomes de carbone, qui est non substitué ou qui est substitué par 1, 2 ou 3 radicaux aryle en (C₆-C₁₂) ;
ainsi que leurs sels physiologiquement acceptables.

2. Composé de formule I selon la revendication 1, dans laquelle
R signifie hydrogène ou un radical acyle comprenant 2-25 atomes de carbone ;
R² signifie un radical acyle comprenant 4-25 atomes de carbone ;
R³ signifie un radical acyle comprenant 10-25 atomes de carbone ; et
R⁴ signifie un radical acyle comprenant 2-25 atomes de carbone ;
ainsi que leurs sels physiologiquement acceptables.

3. Composé de formule (II) 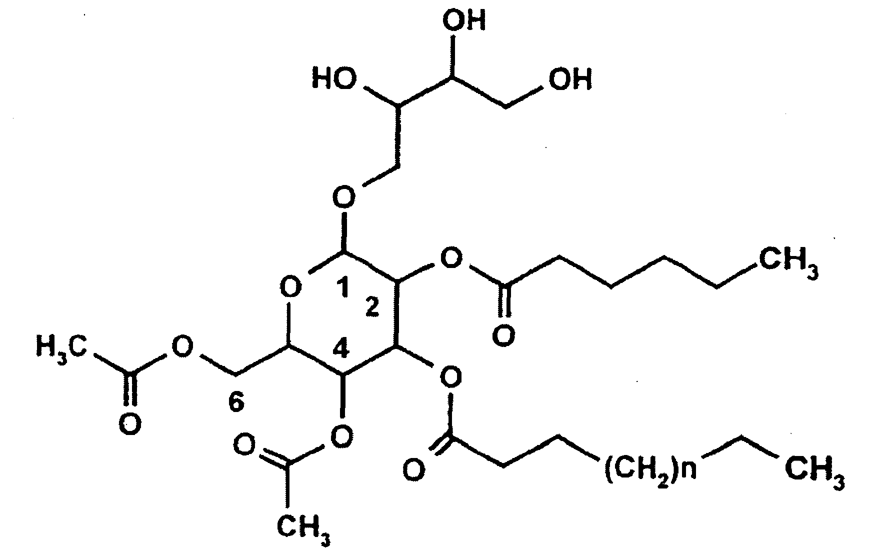 où n vaut 11;
ainsi que ses sels physiologiquement acceptables.

4. Composé de formule (III) 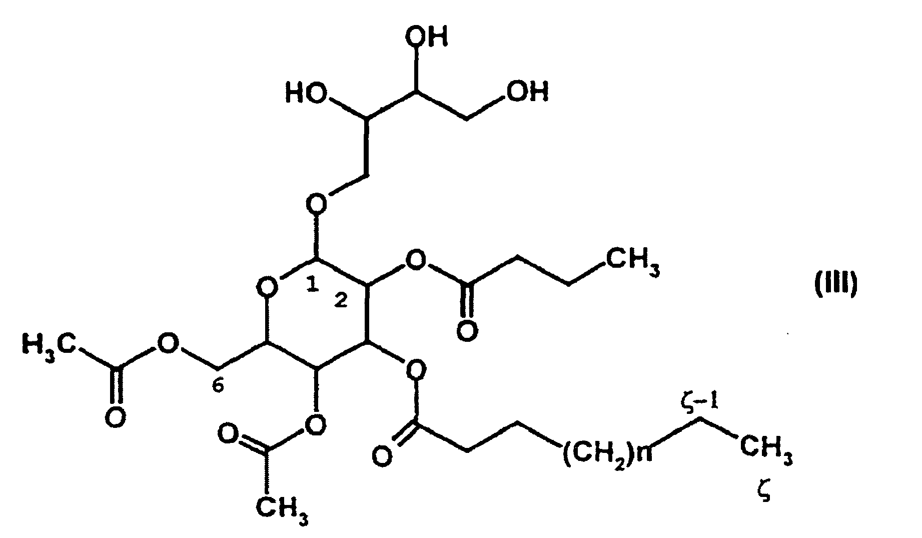 où n vaut 11 ;
ainsi que ses sels physiologiquement acceptables.

5. Composé de formule (IV) 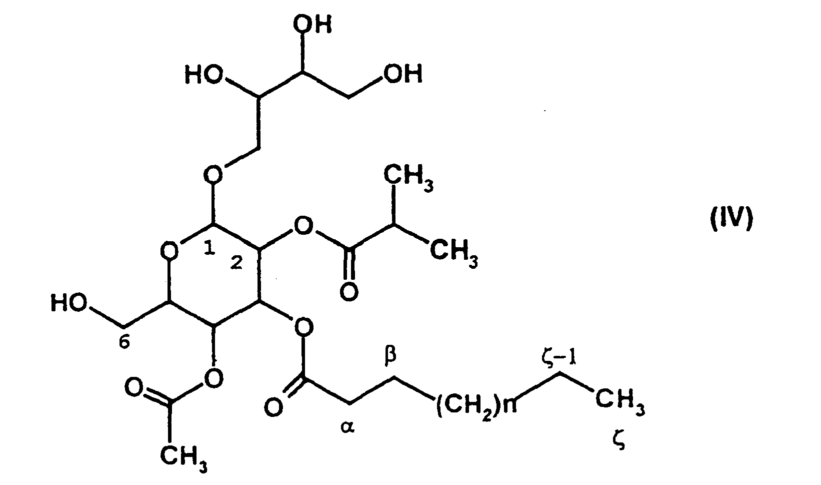 où n vaut 11;
ainsi que ses sels physiologiquement acceptables.

6. Composé de formule I, II, III ou IV ou sel physiologiquement acceptable de celui-ci selon l'une ou plusieurs des revendications 1-5, pouvant être préparé en ce qu'on fermente le microorganisme Ustilago maydis DSM 11494 ou un de ses variants ou mutants dans des conditions appropriées, on isole un ou plusieurs des ustilipides et on les transforme le cas échéant en des sels physiologiquement acceptables.

7. Procédé pour la préparation d'un composé de formule I, II, III ou IV ou d'un sel physiologiquement acceptable de celui-ci selon l'une ou plusieurs des revendications 1-5, **caractérisé en ce qu'**on fermente le microorganisme Ustilago maydis DSM 11494 ou un de ses variants ou mutants dans des conditions appropriées, on isole un ou plusieurs des ustilipides et on les transforme le cas échéant en sels physiologiquement acceptables.

8. Procédé selon la revendication 7, **caractérisé en ce que** la fermentation est réalisée dans des conditions aérobies à une température entre 20 et 35°C et à un pH entre 3 et 10.

9. Composé de formule I, II, III ou IV ou sel physiologiquement acceptable de celui-ci selon l'une ou plusieurs des revendications 1-5 destiné à une utilisation comme médicament.

10. Composé de formule I, II, III ou IV ou sel physiologiquement acceptable de celui-ci selon l'une ou plusieurs des revendications 1-5 destiné à une utilisation comme antagoniste du récepteur de la dopamine D2 et/ou de la dopamine D3.

11. Utilisation d'un composé de formule I, II, III ou IV ou d'un sel physiologiquement acceptable de celui-ci selon l'une ou plusieurs des revendications 1-5 pour la préparation de médicaments destinés au traitement de la schizophrénie et de maladies provoquées par un dysfonctionnement du métabolisme de la dopamine.

12. Médicament présentant une teneur en au moins un composé de formule I, II, III ou IV ou un sel physiologiquement acceptable de celui-ci selon l'une ou plusieurs des revendications 1-5.

13. Procédé pour la préparation de médicaments selon la revendication 12, **caractérisé en ce qu'**on amène au moins un composé de formule I, II, III ou IV ou un sel physiologiquement acceptable de celui-ci selon l'une ou plusieurs des revendications 1-5 avec les adjuvants et/ou supports appropriés en une forme d'administration appropriée.

14. Microorganisme Ustilago maydis DSM 11494.
